# FASCICULE DE BREVET EUROPEEN

(11) **EP 2 116 222 B1**
(45) Date de publication et mention de la délivrance du brevet: **09.05.2018**
(21) Numéro de dépôt: 09004005.6
(22) Date de dépôt: 20.03.2009
(51) Int. Cl.: A61K 8/49, A61Q 19/00, A61Q 19/08

(54) **Utilisation cosmétique de composés conjugués d'auxines indoliques**
Kosmetische Anwendung von indolischen konjugierten Auxinverbindungen
Cosmetic use of compounds combined with indolic auxins

(30) Priorité: 31.03.2008 FR 0801755
(43) Date de publication de la demande: 11.11.2009
(73) Titulaire: Exsymol S.A.M., 98000 Monaco (MC)
(72) Inventeur: Seguin, Marie-Christine, 98000 Monaco (MC)
(74) Mandataire: Macquet, Christophe

(56) Documents cités:
- WO-A-99/00079
- WO-A-03/047558
- FR-A- 1 269 573
- US-A- 5 514 505
- D. E. ZACHARIAS, K. PROUT, C. B. MYERS AND J. P. GLUSKER: "Comparison of the structures of the Plant Growth Hormone Indole-3-acetic Acid, and six of its Amino-Acids Conjugates" INTERNATIONAL UNION OF CHRSTALLOGRAPHY, vol. b47, 1991, pages 107-115, XP002504938 Elsevier

## Description

L'invention concerne la sélection et l'utilisation de composés conjugués d'auxines indoliques dans le domaine cosmétique, précisément comme agent neurotrophique agissant sur le système nerveux cutané.

La presse spécialisée annonçait récemment que « la cosmétique est en pleine frénésie verte » (Cosmétique Magazine, Novembre 2007), évoquant ainsi un segment de marché à l'essor actuellement grandissant, celui des cosmétiques naturels labellisés "bio" issus d'une production biologique et écologique. De surcroît, de récentes campagnes médiatiques alarmistes sur les dangers de certains ingrédients dérivés de la pétrochimie (phtalates, etc) contribuent indirectement à renforcer cette attirance du consommateur pour le "naturel" et les produits dont la proximité avec la nature est simple et sans équivoque.

La présente invention a été développée dans ce contexte, et vise à satisfaire cette demande croissante pour des ingrédients, produits, ou préparations cosmétiques présentant une "naturalité" clairement affichée. Pour cela, la demanderesse a choisi l'option consistant à proposer des composés "naturel-identiques", répliques exactes de substances présentes dans la nature, obtenues par voie chimique.

En choisissant la synthèse chimique plutôt que l'extraction, on obtient une molécule parfaitement pure, ce qui permet d'éviter les problèmes posés par les inévitables impuretés issues de l'extraction de la matière végétale (faible activité, toxicité, instabilité, etc). Cette approche est particulièrement appropriée pour obtenir des composés naturels trouvés dans des plantes rares ou protégées.

La démarche de la demanderesse a consisté à "passer au crible" un vaste ensemble de composés présents dans le règne végétal, et de rechercher des éléments structuraux propres à leurs conférer des propriétés avantageuses, exploitables dans une application cosmétique.

Le choix de la demanderesse s'est arrêté sur les auxines indoliques, composés organiques présents dans toutes les plantes supérieures à graines. Les auxines sont impliquées dans les mécanismes de prolifération et de division cellulaire des plantes (O. Leyser, Current Biology (2001), vol.11, R728). Elles sont indispensables au développement des principaux organes végétatifs : racine, tige et feuille (Davies P.J., In Plant Hormones Physiology, Biochemistry and Molecular Biology, P.J. Davies ed. Kluwer Academic Publishers, pp.1-12).

L'intérêt de la demanderesse s'est porté sur cette classe particulière d'auxines possédant un noyau indole et une chaîne aliphatique alcanecarboxylique. Le noyau indole est en effet une structure hétérocyclique aromatique propre à conférer à la molécule des propriétés antioxydantes, comme l'atteste différentes articles scientifiques (Politi V. et al., Adv. Exp. Med. Biol. (1996), vol.398, pp.291-298 ; Le Borgne M. et al., Ann. Pharm. Fr. (2000), vol.58, pp.316-320). L'auxine acide indole-3-propionique est même annoncée comme l'un des antioxydants naturels les plus puissants (Karbonik J. et al., J. Cellular Biochem. (2001), vol.81, pp.507-513), supérieur en tout cas à la mélatonine et à son homologue acide indole-3-acétique (Chyan Y.J. et al., J. Biol. Chem. (1999), vol.274, pp.21937-21942).

En outre, la demanderesse a, de manière limitative, réduit son intérêt à des conjugués d'auxines présents à l'état naturel, résultant de la conjugaison de l'auxine à la fonction alpha-amine d'un aminoacide via une liaison de type amide, ces conjugués ayant ainsi la formule générale suivante :

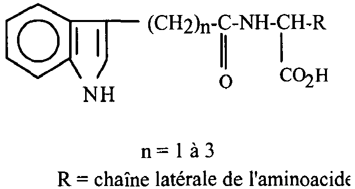

Ces conjugués d'auxines possèdent en effet, à l'inverse de leurs homologues non conjugués, des propriétés physico-chimiques adaptées à un actif cosmétique, à savoir une solubilité dans l'eau, et plus généralement une polarité qui contribue à limiter le passage transcutané, et l'exposition systémique qui résulte du captage par la microcirculation (résorption).

Dans la plante, le rôle de ces conjugués n'est pas entièrement défini, mais il est retenu, en particulier pour les conjugués de l'acide indole-3-acétique, que ce sont des formes de stockage et de transport de l'auxine (Woodward A.W. et al., Annals of Botany (2005), vol.95, pp.707-735, et références citées). Ces conjugués conservent un pouvoir antioxydant, et en particulier un rôle protecteur contre une dégradation péroxydante (Cohen J.D. et al., Annual Rev. Plant Physiol. (1982), vol.33, pp.403-430).

Les vertus antioxydantes de formes conjuguées d'auxines indoliques ont d'ailleurs été déjà exploitées. Tout particulièrement, il est à signaler la demande internationale WO 2005/062851 qui revendique composition pharmaceutique à base de dérivés amides de l'acide indole-3-propionique, et utile dans la prévention ou le traitement de conditions associées à un état oxydatif.

Les répliques chimiques d'un ensemble de conjugués aminoacides d'auxines indoliques ont alors été obtenues par la demanderesse à l'aide de méthodes de synthèse classiques, par le couplage de l'acide indole-3-acétique ("IAA") ou l'un de ses deux homologues, les acides indole-3-propionique ("IPA") et indole-3-butyrique ("IBA"), à différents aminoacides naturels choisis parmi l'alanine, l'arginine, l'acide aspartique, la cystéine, l'acide glutamique, la glycine, l'histidine, la leucine, la lysine, la méthionine, la phénylalanine, la proline, la sérine, le tryptophane, la tyrosine et la valine.

Le criblage systématique des propriétés de chacun de ces composés conjugués synthétisés a ensuite été réalisé. Il a ainsi été confirmé le potentiel antioxydant évoqué ci-avant, avec, pour toutes ces formes conjuguées, une capacité notable, variable cependant selon la nature de l'auxine indolique, à inhiber l'oxydation du désoxyribose dans le système oxydant testé représenté par le couple xanthine oxydase/hypoxanthine.

Par ailleurs, et c'est là le fondement de la présente invention, il a été découvert, pour un très petit panel de structures limitées aux seuls conjugués des acides aspartique et glutamique, une propriété inattendue, à savoir une activité dite neurotrophique, activité également rencontrée chez un ensemble de protéines endogènes, les neurotrophines, et en particulier le "NGF" (ou "nerve growth factor"), un facteur de croissance et de survie essentiel dans la formation et le maintien du réseau nerveux cutané (M. Bothwell, J. Invest. Dermatol. Symposium Proceedings (1997), vol.2, pp.27-30). Cette activité remarquable, sans lien avec les propriétés anti-oxydantes de ces composés, a été mise en évidence à l'aide d'un test *in vitro* consistant à mesurer la cinétique de développement des prolongements de neurones sensitifs issus d'embryons de rats, sélectionnés pour leur physiologie très proche de celle des neurones sensitifs humains.

Au total, cinq conjugués d'auxines résultant du couplage des acides indole-3-acétique, indole-3-propionique et indole-3-butyrique avec successivement les acides aspartique et glutamique ont ainsi montré une activité neurotrophique significative dans ce test.

La peau étant un organe richement innervé par un réseau de fibres nerveuses présent dans l'hypoderme, le derme, et jusque dans l'épiderme (L. Misery, Int. J. Cometic Sc. (2002), vol.24, pp.111-116), l'invention a ainsi pour objet l'utilisation d'un conjugué d'auxine choisi parmi l'acide indolyl-3-acétylglutamique (IAA-Glu), l'acide indolyl-3-propionylglutamique (IPA-Glu), l'acide indolyl-3-propionylaspartique (IPA-Asp), l'acide indolyl-3-butyrylglutamique (IBA-Glu), ou l'acide indolyl-3-butyrylaspartique (IBA-Asp) comme agent neurotrophique pour favoriser l'activité neurotrophique favorable au maintien du réseau nerveux cutané. Le conjugué de formule (I) est choisi parmi les composés acides indolyl-3-acétylglutamique (n=1 et m=2 ; "IAA-Glu") et indolyl-3-butyrylaspartique (n=3 et m=1 ; "IBA-Asp"), préférentiellement le composé acide indolyl-3-butyrylaspartique.

Utilisé avantageusement dans une composition cosmétique, le conjugué de formule (I) décrit ci-avant est présent dans une telle composition en une quantité comprise entre 0,001 et 0,1% % en poids par rapport au poids total de ladite composition, de préférence entre 0,01 et 0,05 % en poids. Cette composition cosmétique vise notamment à préserver les terminaisons nerveuses cutanées.

Puisqu'aujourd'hui est clairement admise l'implication du système nerveux cutané dans différents troubles cutanés liés au vieillissement, tels que des troubles de la desquamation, une sécheresse cutanée, une cicatrisation altérée, un déclin de la fonction sensorielle, etc (L. Misery, Int. J. Cosmetic Sci. (2002), vol.24, pp.111-116), il est particulièrement retenu l'utilisation desdits conjugués d'auxines indoliques de formule (I) dans une composition cosmétique destinée à lutter contre les désordres liés au processus de vieillissement de la peau, et notamment le processus de neurodégénérescence.

Selon un autre mode particulier de l'invention, ladite composition est susceptible de comprendre, en outre, un agent antioxydant tel que des vitamines (C, E, et D), l'acide ascorbique ou encore des composés phénoliques et polyphénoliques (flavonoïdes, etc), ceci afin d'obtenir un effet neuroprotecteur renforcé résultant de la combinaison d'un effet antioxydant et d'un effet neurotrophique.

Cette même composition comprendre également un excipient physiologiquement compatible avec la peau. Elle est enfin adaptée à une administration par voie topique cutanée, présentée sous toutes les formes normalement utilisées pour une telle administration. De manière avantageuse, elle est formulée sous forme, par exemple, d'émulsions, crème, lait, gel, lotion, etc.

Enfin, un procédé de soin cosmétique destiné à préserver les terminaisons nerveuses cutanées est décrit, procédé mis en oeuvre en appliquant sur la peau, éventuellement dans un excipient physiologiquement acceptable et une quantité cosmétiquement efficace d'au moins un conjugué d'auxine indolique tel que défini précédemment est appliqué sur la peau, préférentiellement le composé acide indolyl-3-butyrylaspartique.

L'invention est illustrée par le test suivant, évoqué plus haut dans la description de l'invention.

**Test :** mise en évidence de l'activité neurotrophique des conjugués aminoacides d'auxine indolique

L'étude expérimentale a été réalisée sur des neurones sensitifs dissociés à partir d'explants de ganglions rachidiens issus d'embryons de rats, et préparés selon la méthode décrite par Hall et L. (J. Neurosci. (1997), vol. 17, pp.2775-2784). Après dissection et dissociation, les neurones sensitifs sont immédiatement placés à incubation dans les milieux de culture suivants (en hexaplicate), selon trois configurations :
- cas 1 : milieu de culture sans NGF, et sans ingrédient actif objet de l'invention (contrôle)
- cas 2 : milieu de culture sans NGF, avec un ingrédient actif objet de l'invention
- cas 3 : milieu de culture avec NGF (référence positive)

Après 72 heures d'incubation, les cellules sont lavées puis fixées. Elles sont ensuite marquées par un anticorps anti béta-tubuline réagissant avec les corps cellulaires et les prolongements des neurones. Après la révélation par un anticorps secondaire couplé Alexa Fluor 488, il est procédé à la saisie d'images numérisées en couleurs ("In Cell Analyzer 1000"). Ces données numériques sont ensuite utilisées pour mesurer une densité relative de prolongement par rapport à la condition témoin non traitée (cas 1).

L'effet neurotrophique des composés testés s'exprime par une augmentation (en µm) de cette densité de prolongement des neurones, synonyme d'une cinétique de développement renforcée du réseau neuronal et d'une plus grande viabilité (survie) de celui-ci.

Les résultats sont reportés dans le tableau suivant (sachant que le composé IAA-ASP est hors-invention) :

| **substance** | **concentration (mg/ml)** | **densité moyenne prolongement neurones sensitifs (*µ*m)** | **% par rapport au contrôle** |
|---|---|---|---|
| contrôle (cas 1) | - | 6284 | - |
| IAA-Glu (cas 2) | 0,12 | 7335 | 117 |
| IAA-Asp (cas 2) | 0,12 | 6820 | 108 |
| IPA-Glu (cas 2) | 0,12 | 8247 | 131 |
| IPA-Asp (cas 2) | 0,12 | 6980 | 111 |
| IBA-Glu (cas 2) | 0,12 | 7195 | 114 |
| IBA-Asp (cas 2) | 0,12 | 8297 | 132 |
| témoin NGF (cas 3) | 5.10⁻⁶ | 11750 | 187 |

## Revendications

1. Utilisation cosmétique d'un conjugué d'auxine choisi parmi l'acide indolyl-3-acétylglutamique (IAA-Glu), l'acide indolyl-3-propionylglutamique (IPA-Glu), l'acide indolyl-3-propionylaspartique (IPA-Asp), l'acide indolyl-3-butyrylglutamique (IBA-Glu), ou l'acide indolyl-3-butyrylaspartique (IBA-Asp) comme agent neurotrophique pour favoriser l'activité neurotrophique favorable au maintien du réseau nerveux cutané.

2. Utilisation selon la revendication 1, **caractérisée en ce que** ledit conjugué est choisi parmi les composés acides indolyl-3-acétylglutamique et indolyl-3-butyrylaspartique.

3. Utilisation selon l'une des revendications 1 ou 2, **caractérisée en ce que** ledit conjugué est le composé acide indolyl-3-butyrylaspartique.

4. Utilisation selon l'une quelconque des revendications précédentes, **caractérisée en ce que** ledit conjugué est présent en une quantité comprise entre 0,001 et 0,1% % en poids d'une composition cosmétique par rapport au poids total de ladite composition, de préférence entre 0,01 et 0,05 % en poids.

5. Utilisation selon la revendication 4, **caractérisée en ce que** ladite composition comprend, en outre, un agent antioxydant.

6. Utilisation selon la revendication 5, **caractérisée en ce que** ledit agent antioxydant est choisi parmi des vitamines, l'acide ascorbique et des composés phénoliques et polyphénoliques.

7. Utilisation selon l'une quelconque des revendications 4 à 6, **caractérisée en ce que** ladite composition comprend un excipient physiologiquement compatible avec la peau et **en ce qu'**elle est d'application topique.

## Patentansprüche

1. Kosmetische Verwendung eines Auxin-Konjugats, ausgewählt aus Indolyl-3-acetylglutaminsäure (IAA-Glu), Indolyl-3-propionylglutaminsäure (IPA-Glu), Indolyl-3-propionylasparaginsäure (IPA-Asp), Indolyl-3-butyrylglutaminsäure (IBA-Glu), oder Indolyl-3-butyrylasparaginsäure (IBA-Asp) als neurotroper Wirkstoff, um die neurotrope Aktivität, die den Erhalt des Hautnervengeflechts unterstützt, zu fördern.

2. Verwendung nach Anspruch 1, **dadurch gekennzeichnet, dass** das Konjugat aus den Indolyl-3-acetylglutaminsäure- und Indolyl-3-butyrylasparaginsäure-Verbindungen ausgewählt ist.

3. Verwendung nach einem der Ansprüche 1 oder 2, **dadurch gekennzeichnet, dass** das Konjugat die Indolyl-3-butyrylasparaginsäure-Verbindung ist.

4. Verwendung nach einem der vorstehenden Ansprüche, **dadurch gekennzeichnet, dass** das Konjugat in einer Menge im Bereich zwischen 0,001 1 und 0,1 % Ges.-% einer kosmetischen Zusammensetzung bezogen auf das Gesamtgewicht der Zusammensetzung, vorzugweise zwischen 0,01 und 0,05 Gew.-% vorliegt.

5. Verwendung nach Anspruch 4, **dadurch gekennzeichnet, dass** die Zusammensetzung weiter ein Antioxidationsmittel umfasst.

6. Verwendung nach Anspruch 5, **dadurch gekennzeichnet, dass** das Antioxidationsmittel ausgewählt ist aus Vitaminen, Ascorbinsäure und Phenol- und Polyphenolverbindungen.

7. Verwendung nach einem der Ansprüche 4 bis 6, **dadurch gekennzeichnet, dass** die Zusammensetzung einen physiologisch hautverträglichen Exzipienten umfasst und dadurch, dass sie von topischer Anwendung ist.

## Claims

1. Cosmetic use of an auxin conjugate selected from the indolyl-3-acetylglutamic acid (IAA-Glu), the indolyl-3-proprionylglutamic acid (IPA-Glu), the indolyl-3-propionylaspartic acid (IPA-Asp), the indolyl-3-butyrylglutamic acid (IBA-Glu), or the indolyl-3-butyrylaspartic acid (IBA-Asp) as neurotrophic agent in order to promote the neurotrophic activity responsible for maintaining the cutaneous nervous network.

2. Use according to claim 1, **characterized in that** said conjugate is selected from the indolyl-3-acetylglutamic and indolyl-3-butyrylaspartic acid compounds.

3. Use according to claim 1 or 2, **characterized in that** said conjugate is the indolyl-3-butyrylaspartic acid compound.

4. Use according to one of the preceding claims, **characterized in that** said conjugate is present in an amount comprised between 0.001 and 0.1% by weight of a cosmetic composition in relation to the total weight of said composition, preferably between 0.01 and 0.05% by weight.

5. Use according to claim 4, **characterized in that** said composition further comprises an antioxidant.

6. Use according to claim 5, **characterized in that** said antioxidant is selected from vitamins, ascorbic acid, phenolic and polyphenolic compounds.

7. Use according to one of the claims 4 to 6, **characterized in that** said composition comprises an excipient physiologically compatible with the skin and **in that** it is for topical application.
